(19)

Europäisches
Patentamt

European
Patent Office

Office européen
des brevets

(11) **EP 2 772 757 A1**

(12) **EUROPEAN PATENT APPLICATION**

(43) Date of publication:
**03.09.2014 Bulletin 2014/36**

(51) Int Cl.:
**G01N 33/49** (2006.01)     **G01N 31/22** (2006.01)
**G01N 33/18** (2006.01)

(21) Application number: **13425030.7**

(22) Date of filing: **28.02.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Biotoxen Srl**
**73100 Lecce (IT)**

(72) Inventor: **Biotoxen Srl**
**73100 Lecce (IT)**

(74) Representative: **Bruni, Giovanni**
**Laforgia, Bruni & Partners
Corso Duca degli Abruzzi, 78
10129 Torino (IT)**

<u>Remarks:</u>
Amended claims in accordance with Rule 137(2)
EPC.

(54) **Colorimetric method and apparatus for measuring the toxic effect of environmental pollutants on hemoglobin**

(57)     Method for measuring the toxicity of environmental matrices characterized in that it uses the oxidation of the iron present in the hemoglobin molecule as indicator of toxicity of said environmental matrices according to the steps of adding hemoglobin in an aqueous sample of environmental matrix, adding potassium thiocyanate, quantifying the oxidation state of the iron present in the hemoglobin molecule by means of colorimetric analysis of the sample.

EP 2 772 757 A1

## Description

Object of the present invention is a new method for evaluating and measuring toxicity of environmental matrices. Said method is specifically referred to a test based on the hemoglobin oxidation in response to different classes of contaminants present in environmental matrices, such for example, waters, elutriats, percolates, sewages, etc...

Generally, with "environmental contamination" is defined the consequence of a human action able to modify the properties of the conditions or the availability or the quality of resources in a determined interval of space and time (Vighi and Bacci, 1988) where with "resources" it is intended those components of the natural systems, both of biotic and abiotic origin, which are normally exploited by organisms for their survival; and with "conditions" it is intended the group of abiotic environmental factors which are not consumed by the living organisms (temperature, water medium transparency etc...). The environmental contamination becomes then "pollution" when it causes "measurable" damages to a biological system, such as a cellular or tissue biochemical process, or to an organism, up to the alterations of a population and a biological community (Vighi and Bacci, 1988). One usually speaks about water, air, soil and food pollution and the method, object of the present invention, is useful and can be used to determine the quality criteria and the concentration limits of such pollutants. Recently, it has became always more interesting the risk evaluation associated to the presence of environmental matrices of toxic substances. Always more increasing is the research for methodologies of rapid, simple and economic investigation as well for screening the presence of toxicity in environmental matrices, methodologies which can be advantageous both from the economic and technical point of view for operators and professionals operating in the environmental and food field.

At the state of the art, there are known methods and systems for detecting hemoglobin as molecule in toto. Said methods and systems refer mostly to the medical and pharmaceutical fields and therefore are not specific for the evaluation of the toxicity of the environmental matrices. It is very innovative the application of the hemoglobin molecule as "biomolecular detector" of the presence of toxic substances in environmental samples. In fact, currently there are not known methods based on the oxidation of the hemoglobin as analysis element of the environmental toxicity.

Therefore, aim of the present invention is to provide a measure of the toxicity level of a determined sample of environmental matrix by measuring the oxidation of the hemoglobin molecule. The sample to be analyzed has to be in aqueous form and therefore, according to the matrix to be analyzed, there will be used methods to reach the aqueous sample to be used. For example, in case of solid matrices as soil or sediment, the interstitial water will be extracted from the matrix by means of centrifugation of the sample and/or elutriat by means of incubation of the sample with synthetic water (fresh water or sea water according to the starting matrix) and subsequent centrifugation.

The method, object of the invention is to be considered a first test for a rapid environmental screening, simple to be used and economic, and apt to evaluate the toxicity of different kinds of environmental matrices.

As it is known, hemoglobin is a globular protein whose quaternary structure is made up of 4 subunits; it is soluble, of red color (it is a chromoprotein) and it is present in the red blood cells of vertebrates, with the exception of some antarctic fishes; it is responsible for the transport of molecular oxygen from a compartment with high $O_2$ concentration to the tissues needing it. Each one of its four proteic globules, called globin, has inside a protoporphyrin molecule which coordinates an iron ion Fe (II), positioned lightly outside the molecule plane, in the group called Heme group.

The hemoglobin can be oxidized physiologically or for effect of determined oxidant substances of organic and inorganic kind (metal cations, nitrites, nitrates, derivatives of toluene and benzene): the iron ion (II) (ferrous ion) turns into iron ion (III) (ferric ion) ($Fe^{3+}$) and the hemoglobin turns into methemoglobin (MetHb) with the oxygen linked to iron by means of a strong link (covalent) which makes it not available to the tissues. The change of the oxidation state deprives the molecule of its capacity to link the oxygen reversibly, and so of its physiological function of transport.

According to what referred in literature, the concentration of methemoglobin can be determined by means of various methods as for example 8-wavelength pulsed oximeter (Barker et al., Anesthesiology, 105: 892-897), cooximeters, or by means of the colorimetric method described by Evelyn and Malloy (1938) based on the usage of potassium cyanide (KCN) and potassium ferrocyanide ($K_3Fe(CN)_6$). The potassium ferrocyanide oxidizes the hemoglobin species in methemoglobin, the potassium cyanide links itself to $Fe^{3+}$ of the methemoglobin forming the cyanomethemoglobin, a very stable red compound. The concentration of methemoglobin is obtained for difference from the value obtained measuring the absorbance of the sample at 633 nm in presence and in absence of $K_3Fe(CN)_6$.

The present invention is innovative not only for the application of the hemoglobin oxidation measuring as detection means of toxicity of environmental water matrices, but also for the method of hemoglobin measuring in the oxidized state. The concentration of methemoglobin present in the sample is measured by means of an innovative colorimetric analysis method based only on the usage of potassium thiocyanate, excluding the combined use of more substances such for example potassium cyanide and potassium ferrocyanide, as in the known colorimetric methods. The use of the sole thiocyanate implies many advantages with respect to the combined use of potassium cyanide and potassium ferrocyanide as it is described in the standard method:

- more safety for the operator since it is avoided the use of cyanide, a highly toxic substance, in favour of the thiocyanate, a much less toxic substance;
- simpler operation of the method because for each sample it is not needed to carry out the double measure with and without the potassium ferrocyanide;
- reduction of the analysis costs since it is used only a compound, the thiocyanate.

Specifically, the method object of the present invention comprises the use of purified hemoglobin (for example mammalian purified hemoglobin), available in the market, which is put in contact with environmental samples whose toxicity it is desired to be tested.

The hemoglobin is prepared in an aqueous solution in a concentration hemoglobin/dilled water 1mg/ml. The aqueous sample to be tested, taken from environmental matrices, is arranged in a suitable container, for example a cuvette, in contact with the hemoglobin dissolved in aqueous solution, preferably in a proportion 1:1 (for example $100\mu l$ dissolved hemoglobin and $100\mu l$ sample). After a certain time interval, preferably up to 30 minutes, the potassium thiocyanate in aqueous solution is added to the reaction mixture (preferably 40 mM). Specifically, the potassium thiocyanate reacts with the iron ion ($Fe^{3+}$) of the methemoglobin, oxidized by toxic substances possibly present in the sample, and causes the formation in the reaction mixture of a red color chromogen complex (having an absorbance peak at the wavelenght of 480 nm).

After an incubation interval of 10 minutes, needed for the chromogen compound to develop, the optic density of the sample is read spectrophotometrically at the wavelenght of 480nm. The higher the oxidation entity of the iron ion contained in the hemoglobin is, the higher the value of the optic density measured will be and the higher the toxicity level of the analyzed environmental sample will be. The toxicity of the sample is measured as percent of effect with respect to a control sample realized in absence of the environmental sample, positioning only the hemoglobin and the potassium thiocyanate in the cuvette. The percent of effect is calculated according to the present formula:

$$E = (Dot/Doc)*100$$

where E represents the percent of effect, Dot is the optic density measured in cuvette in presence of the sample to be tested and Doc is the optic density of the control test. In order to determine the toxicity of an environmental aqueous sample the sample can be firstly tested as it is. If the percent of effect is up to 20%, the sample can be considered not toxic. If the percent of effect is in a range between 20 and 50% the sample can be considered lightly toxic. If the percent of effect is higher then 50% the sample is defined toxic and it is possible to proceed with a second test carried out on increasing dilutions (at least five, for example, 100%, 50%, 25%, 12.5%, 6.25%) of the sample. The measure of the percent of effect exerted by the different dilutions (always calculated referring to the control sample) allows to draw a semilogarithmic graph in which on the ordinate axis is indicated the percent of effect and on the abscissa axis the logarithm of the concentration of the environmental aqueous sample. By curve interpolation it is possibile to calculate the EC50 value, i.e. the concentration of environmental sample which determines the oxidation of the 50% of the hemoglobin present in the cuvette. The lower the EC50 value is, the higher the toxicity of the analyzed sample will be.

The toxicity test based on the oxidation level of the hemoglobin is resulted sensible both to organic and inorganic chemical pollutants (heavy metals). Concerning the heavy metals, in the tested concentration ranges ($1\mu g/l$ - 1mg/l) it is observed the following scale of test sensibility:

Hg > Cu > Zn > Cd > Ni > Pb

For example in the case of mercury, at the concentration 0,1mg/l the percent of effect results 100%.

Concerning the organic chemical pollutants, it was tested the sensibility to polychlorinated biphenyls (test substance used arochlor), carbamate pesticides (test substance used carbaryl) and polycyclic aromatic hydrocarbons (test substance used benzopyrene). In the tested concentration range (0,01 - $1\mu g/l$) the sensibility scale of test results the following:

arochlor > carbaryl > benzopyrene

For the test application.

Said colorimetric method can be simply used in portable devices as disposable strips made up of a support strip in which there are immobilized the constituents of the invention, put in contact with the environmental aqueous samples (waters, sewages, percolates, elutriats etc...) for a rapid determination of the toxicity without recourse to the analysis of specialized labs. In case of disposable strips, it is used a visible chromatic scale from no color to red (no color, yellow, orange, red): the more intense the coloration tending to red is, the more toxic the sample is.

## Claims

1. Method for measuring the toxicity of environmental matrices **characterized in that** it uses the oxidation of the iron present in the hemoglobin molecule as indicator of toxicity of said environmental matrices according to the following steps:

    - adding hemoglobin in an aqueous sample of environmental matrix;
- adding potassium thiocyanate;
- quantifying the oxidation state of the iron present in the hemoglobin molecule by means of colorimetric analysis of the sample.

**2.** Method according to claim 1, wherein the aqueous sample of environmental matrix is obtained by means of centrifugation.

**3.** Method according to claim 2, wherein said centrifugation is preceded by the incubation of the sample of environmental matrix with synthetic water.

**4.** Method according to any one of the preceding claims, wherein said hemoglobin is dissolved in aqueous solution in a proportion 1:1.

**5.** Method according to any one of the preceding claims, wherein said step of adding hemoglobin in the taken sample lasts less than 30 minutes.

**6.** Method according to any one of the preceding claims, wherein said step of quantifying the oxidation state of the hemoglobin is carried out 10 minutes after the step of adding potassium thiocyanate.

**7.** Method according to any one of the preceding claims, wherein said step of quantifying the oxidation state of the hemoglobin by means of colorimetric analysis is carried out by means of a spectrophotometer.

**8.** Portable device comprising a plurality of disposable strips, which provides the proposed method according to any one of claims 1 to 7.

**Amended claims in accordance with Rule 137(2) EPC.**

**1.** Method for measuring the toxicity of environmental matrices according to the following steps:

- adding hemoglobin in an aqueous sample of environmental matrix;
- adding potassium thiocyanate;
- quantifying the oxidation state of the iron present in the hemoglobin molecule by means of colorimetric analysis of the sample, **characterized** the oxidation of the iron present in the hemoglobin molecule is used as an indicator of toxicity of said environmental matrices.

**2.** Method according to claim 1, wherein the aqueous sample of environmental matrix is obtained by means of centrifugation.

**3.** Method according to claim 2, wherein said centrifugation is preceded by the incubation of the sample of environmental matrix with synthetic water.

**4.** Method according to any one of the preceding claims, wherein said hemoglobin is dissolved in aqueous solution in a proportion 1:1.

**5.** Method according to any one of the preceding claims, wherein said step of adding hemoglobin in the taken sample lasts less than 30 minutes.

**6.** Method according to any one of the preceding claims, wherein said step of quantifying the oxidation state of the hemoglobin is carried out 10 minutes after the step of adding potassium thiocyanate.

**7.** Method according to any one of the preceding claims, wherein said step of quantifying the oxidation state of the hemoglobin by means of colorimetric analysis is carried out by means of a spectrophotometer.

**8.** Portable device comprising a plurality of disposable strips, in which there are immobilized hemoglobin and potassium thiocyanate for a method according to any of claims 1 to 7.

**EUROPEAN SEARCH REPORT**

Application Number

EP 13 42 5030

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (IPC) |
| A | BURDICK B A ET AL: "A MULTILAYER ELEMENT FOR DETERMINING HEMOGLOBIN IN WHOLE BLOOD PRINCIPLES AND ANALYTICAL PERFORMANCE", CLINICAL CHEMISTRY, vol. 32, no. 10, 1986, pages 1953-1955, XP002706424, ISSN: 0009-9147 * the whole document * | 1-8 | INV. G01N33/49 G01N31/22 G01N33/18 |
| A | WO 2009/005884 A1 (3M INNOVATIVE PROPERTIES CO [US]; ZHANG YIFAN [US]; WRIGHT ROBIN E [US]) 8 January 2009 (2009-01-08) * the whole document * | 1-8 | |
| A | US 2003/082076 A1 (LIN YUEH-HUI [TW] ET AL) 1 May 2003 (2003-05-01) * the whole document * | 1-8 | |
| | | | TECHNICAL FIELDS SEARCHED (IPC) |
| | | | G01N |

The present search report has been drawn up for all claims

| Place of search | Date of completion of the search | Examiner |
|---|---|---|
| The Hague | 26 July 2013 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 42 5030

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2013

| Patent document cited in search report | | Publication date | Patent family member(s) | Publication date |
|---|---|---|---|---|
| WO 2009005884 | A1 | 08-01-2009 | NONE | |
| US 2003082076 | A1 | 01-05-2003 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BARKER et al.** *Anesthesiology,* vol. 105, 892-897